# EUROPÄISCHE PATENTSCHRIFT

(11) **EP 2 273 929 B1**
(45) Veröffentlichungstag und Bekanntmachung des Hinweises auf die Patenterteilung: **27.08.2014**
(21) Anmeldenummer: 10722312.5
(22) Anmeldetag: 25.05.2010
(51) Int. Cl.: A61B 17/068, A61B 17/122, A61B 17/128, A61B 17/10, A61B 17/00

(54) **VORRICHTUNG ZUM APPLIZIEREN EINES MEDIZINISCHEN VERRIEGELBAREN CLIPS IN EINEM GEWEBEBEREICH**
DEVICE FOR APPLYING A MEDICAL LOCKABLE CLIP IN A TISSUE AREA
DISPOSITIF POUR APPLIQUER UNE PINCE MÉDICALE VERROUILLABLE DANS UNE PARTIE D'UN TISSU

(30) Priorität: 26.05.2009 DE 102009022692
(43) Veröffentlichungstag der Anmeldung: 19.01.2011
(73) Patentinhaber: Joimax GmbH, 76227 Karlsruhe (DE); Karlsruher Institut für Technologie, 76131 Karlsruhe (DE)
(72) Erfinder: KNEIFEL, Bernhard, 76767 Hagenbach (DE); KÖRBER, Rainer, Dr., 67346 Speyer (DE); HERRMANN, Günter, Dr., 67269 Grünstadt (DE); NOTHEIS, Ekatarina, 63517 Rodenbach (DE); RIES, Wolfgang, 76351 Linkenheim (DE); BRHEL, Klaus-Peter, 76661 Philippsburg (DE)
(74) Vertreter: Lempert, Jost
(86) Internationale Anmeldenummer: PCT/EP2010/003166
(87) Internationale Veröffentlichungsnummer: WO 2010/136170

(56) Entgegenhaltungen:
- EP-A1- 1 199 990
- US-A- 4 569 469
- US-A- 5 425 489
- US-A1- 2006 247 643

## Beschreibung

Die Erfindung betrifft eine Vorrichtung zum Applizieren eines medizinischen verriegelbaren Clips in einem Gewebebereich nach dem Oberbegriff des Anspruchs 1.

Eine derartige Vorrichtung ist aus der EP 1 199 990 B1 bekannt. Nachteilig bei der bekannten Vorrichtung ist, dass die Kraft zum Einschießen und Schließen des Clips manuell aufgebracht werden muss und daher der Vorgang nicht zuverlässig zu reproduzierbaren identischen Ergebnissen führt; zum Teil auch ein schlechtes Setzen des Clips erfolgen kann.

Der Erfindung liegt daher die Aufgabe zugrunde, eine Vorrichtung der eingangs genannten Art zu schaffen, die bzw. das ein zuverlässiges und reproduzierbares Setzen derartiger Clips in Körpergewebe ermöglicht.

Erfindungsgemäß wird die genannte Aufgabe durch eine gattungsgemäße Vorrichtung mit den Merkmalen des Anspruchs 1 gelöst.

Eine automatische Vorrichtung ist eine solche, die vorbestimmte. Handlungen nach einem Auslöseimpuls selbstständig und zwangsläufig ablaufen lässt. Demgemäß ist ein automatisches Verfahren ein solches, bei dem vorbestimmte Handlungen nach einem Auslöseimpuls selbstständig und zwangsläufig ablaufen. Dies kann insbesondere motorisch geschehen, wobei verschiedene motorische Antriebsarten eingesetzt werden können, wie pneumatische, hydraulische, elektrische oder auch elektromagnetische.

Durch die Erfindung wird erreicht, dass der Clip in ein bestimmtes Gewebe reproduzierbar mit gleicher Kraft eingeschossen und geschlossen werden kann, so dass auch gleiche reproduzierbare Ergebnisse erzielt werden. Darüber hinaus wird es möglich, bei unterschiedlich festen oder harten Geweben vorbestimmte unterschiedliche Einschusskräfte einzusetzen, um so auch unabhängig von der Konsistenz des Gewebes eine zuverlässige Festlegung des Clips zu erreichen. Darüber hinaus kann durch die Erfindung sowohl eine größere Einschussgeschwindigkeit als auch eine größere Schließkraft aufgewendet werden, als dies manuell ausführbar ist, wodurch ebenfalls die Festlegung des Clips verbessert wird. Die Vorrichtung kann für den Clip eine formschlüssige und/oder reibschlüssige Aufnahme aufweisen, wobei der Verriegelungsmechanismus ein Kniehebelelement aufweist, der die Greifzähne des Clips gegeneinander drückt und die formschlüssige und/oder reibschlüssige Aufnahme des Clips löst. Eine bevorzugte Ausgestaltung sieht vor, dass die Aufnahme den Clip bevorzugt formschlüssig hält, indem seitlich am Clip bzw. an dessen Zangenflanken Noppen vorgesehen sind, die zur Arretierung des Clips in entsprechende Noppen oder Vertiefungen, welche in der Clipaufnahme ausgebildet sind, greifen. Der Formschluss ist durch die Formänderung des Clips bei Betätigen des Kniehebelmechanismus lösbar.

Alternativ ist bevorzugt vorgesehen, dass die Clipaufnahme den Clip reibschlüssig in der Clipaufnahme selbst oder durch einen Pressgleitsitz zwischen einer möglichen oberen und einer unteren Führung in der Clipaufnahme hält.

Bevorzugte Ausgestaltungen der Erfindung sehen vor, dass der Vorschubmechanismus durch einen Einschusszylinder und das Verriegelungselement durch einen Schließkolben gebildet wird und/oder in einer Zylinderlaufbuchse der Einschusszylinder läuft, in welchem der Schließkolben dichtend geführt ist.

Darüber hinaus kann als alternative Ausgestaltung vorgesehen sein, dass der Einschusszylinder und der Schließzylinder über einen T-förmigen Verteiler und eine Druckleitung verbunden sind, wobei in der Druckleitung zum Schließzylinder ein Reduzierstück angeordnet ist, oder durch eine zwischen Vorschubmechanismus und Verriegelungselement angeordnete, letztere beaufschlagende Feder verbunden sind.

Dadurch wird sowohl der Einschusszylinder als auch der Schließzylinder gleichzeitig mit Druck und gleichem Druckwert beaufschlagt. Beim Bewegen des Vorschubmechanismus wird zunächst der Einschusszylinder bis zum oben erwähnten Anschlag vorgeschoben und schiebt damit den Schließzylinder nach vorn. Der beaufschlagte Druck wird durch den reduzierten Leitungsquerschnitt des Reduzierstücks und die Druckleitung bestimmter Länge zum Schließzylinder langsam ansteigend auf den Schließkolben beaufschlagt und der Verriegelungsmechanismus somit zeitversetzt weiterbewegt, wodurch der Verriegelungsmechanismus mit einer zeitlichen Differenz zum Vorschubmechanismus auf den Kniehebel einwirkt.

Hierdurch wird zuverlässig erreicht, dass zunächst der Clip in das Körpergewebe eingeschossen und es anschließend durch Bewegen der Zähne aufeinander geschlossen wird.

Weitere bevorzugte Ausgestaltungen der Erfindung sehen vor, dass das Verriegelungselement eine Führung für den Clip aufweist und ein Rückholmittel für den pneumatischen Vorschubmechanismus und das Verriegelungselement in die Ausgangsposition, wobei insbesondere die Rückholmittel pneumatische Rückholkolben umfassen.

Erfindungsgemäß folgt auf eine manuelle Ventilbetätigung eine pneumatisch gesteuerte Auslösung des Verfahrens. Dagegen ist in bevorzugter Ausführungsform der Auslösemechanismus als eine Anordnung bestehend aus einem Betätigungsventil, einem Hilfszylinder und einem Öffnungsventil ausgebildet, wobei das Öffnen der Druckluftzufuhr erst nach Erreichen eines vorgegebenen Öffnungsdruck erfolgt. Dies gewährleistet eine Durchführung des Verfahrens mit stets gleichem Druck und gleicher Vorschubbewegung des Clips.

In einer alternativen Ausgestaltung des pneumatischen Vorschubmechanismus kann bevorzugt vorgesehen sein, dass der Einschusskolben innerhalb des Einschusszylinders während des Auslösens nach einem bestimmten Vorschubweg eine Druckverbindung von der Wandung des Einschusszylinders zum Schließzylinder freigibt. Auf diese Weise erfolgt eine zeitversetzte Druckbeaufschlagung des Schließkolbens zum Einschusskolben ohne eine separate Druckluftleitung und/oder einen T-Verteiler.

Darüber hinaus kann erfindungsgemäß ein Clipmagazin mit einer seriellen Clipzuführung in der Ausgangsposition vorgesehen sein. Bevorzugt sieht die Erfindung hierbei vor, dass seitlich vom Schließkolben eine Mehrzahl in distaler Richtung ausgerichteter Clips einsetzbar sind, wobei die Clips hintereinander angeordnet sind. Die Clips sind bevorzugt vom proximalen Ende eines möglichen Magazins durch Vorschubmittel, wie z.B. eine Feder, in distaler Richtung gegen einen distalen Anschlag an der Clipaufnahme vorschiebbar. Nach einer Applikation eines Clips erfolgt ein Zurückziehen des Schließkolbens und bevorzugt auch der Clipaufnahme, wobei der Clip im Magazin, welcher dem distalen Anschlag an der Clipaufnahme am nächsten ist, in die Clipaufnahme eingeschoben - durch z.B. eine Blattfeder - und der folgende Clip im Magazin an den distalen Anschlag geschoben wird.

Weiterhin sieht die Erfindung eine Sicherung gegen ungewolltes Auslösen des automatischen Ablaufs vor.

Weitere Vorteile und Merkmale der Erfindung ergeben sich aus den Ansprüchen aus der nachfolgenden Beschreibung, in der ein Ausführungsbeispiel der Erfindung unter Bezugnahme auf die Zeichnung im Einzelnen erläutert ist. Dabei zeigt:
- Fig. 1: einen Clip in Ausgangskonfiguration;
- Fig. 2: den Clip in der gleichen Konfiguration mit durch Punkte angedeutete Filmscharniere oder -gelenke;
- Fig. 3: den Clip bei mittiger axialer Beaufschlagung des Kniehebels unmittelbar vor Umschlag in die stabile Endlage;
- Fig. 4: den Clip in seiner stabilen Endlage mit überstrecktem Kniehebel;
- Fig. 5: eine schematische Darstellung zu der Form der erfindungsgemäßen Vorrichtung in Ausgangsstellung und Vorschubmechanismus in Bereitschaftsstellung;
- Fig. 6: eine schematische Darstellung der erfindungsgemäßen Vorrichtung gemäß Fig. 5 in zueinander entgegen gesetzter Ansicht;
- Fig. 7: eine schematische Darstellung des distalen Endes der Vorrichtung einer alternativen Ausführungsform mit eingesetztem Clip nach Schnitt A-A der Fig. 6;
- Fig. 8: eine weitere schematische Darstellung der Vorrichtung gemäß Fig. 5 mit teilweise vorgeschobenen Vorschubmechanismus;
- Fig. 9: eine schematische Teildarstellung des vorderen Bereichs der erfindungsgemäßen Vorrichtung gemäß Fig. 5 mit vorgeschobenen Verriegelungselementes (Schließkolben);
- Fig. 10: einen Pneumatikplan nach DIN/ISO 1219 einer erweiterten Ausgestaltung der erfindungsgemäßen Vorrichtung.

Der erfindungsgemäß eingesetzte - resorbierbare - Clip 1 weist mittig eine verformbare Schulter 1.1 auf, auf dessen einen Seite an den Endbereichen jeweils ein Zahn 1.2 mit einer scharfen Spitze 1.3 ausgebildet ist.

Auf der den Zähnen 1.2 gegenüberliegenden Seite der Schulter 1.1 ist ebenfalls einstückig ein Kniehebel 1.4 ausgebildet, der über Filmgelenke 1.5 mit der Schulter 1.1 verbunden ist und dessen beiden Teile ebenfalls mittig über ein Filmgelenk 1.6 miteinander verbunden sind.

Unterhalb des Kniehebels 1.4 weist die Schulter eine Ausnehmung 1.7 auf, in die der Kniehebel 1.4 in Überstreckstellung eindrückbar ist.

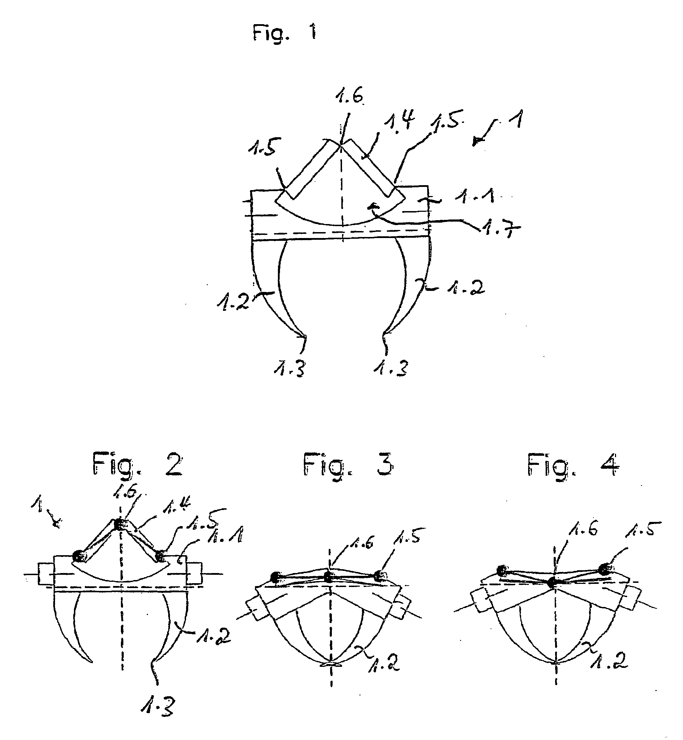

Die Fig. 1 und 2 zeigen den Clip 1 in seiner unbelasteten Ausgangs- oder Bereitschaftsstellung mit geöffneten Zähnen 1.3. Die Fig. 3 zeigt eine Konfiguration des Clips 1, bei dem dieser mittig auf die beiden im Filmgelenk 1.6 einander berührenden Enden der Teile des Kniehebels 1.4 mit einer Kraft beaufschlagt wird, während auf die Enden der Schulter 1.1 eine Gegenkraft wirkt. Hierdurch werden die Zähne 1.2, wie dies in Fig. 3 dargestellt ist, geschlossen. Durch Überdehnen mittels der einwirkenden Kraft entsprechend der Konfiguration der Fig. 4 gelangt der Kniehebel 1.4 in eine stabile Position, bei der dieser die Schließstellung des Clips 1 auch ohne weitere Krafteinwirkungen auf seinen Mittelbereich aufrechterhält.

Die erfindungsgemäße Vorrichtung führt die folgenden Schritte nacheinander aus:
- Vorschub des Clips 1 in der Ausgangs- oder Bereitschaftskonfiguration gemäß Fig. 1 und 2 unter Einstechen der Spitzen 1.3 der Zähne 1.2 in ein Gewebe;
- folgendes Schließen des Clips 1 oder der Klammer entsprechend den Konfigurationen der Fig.3 und 4

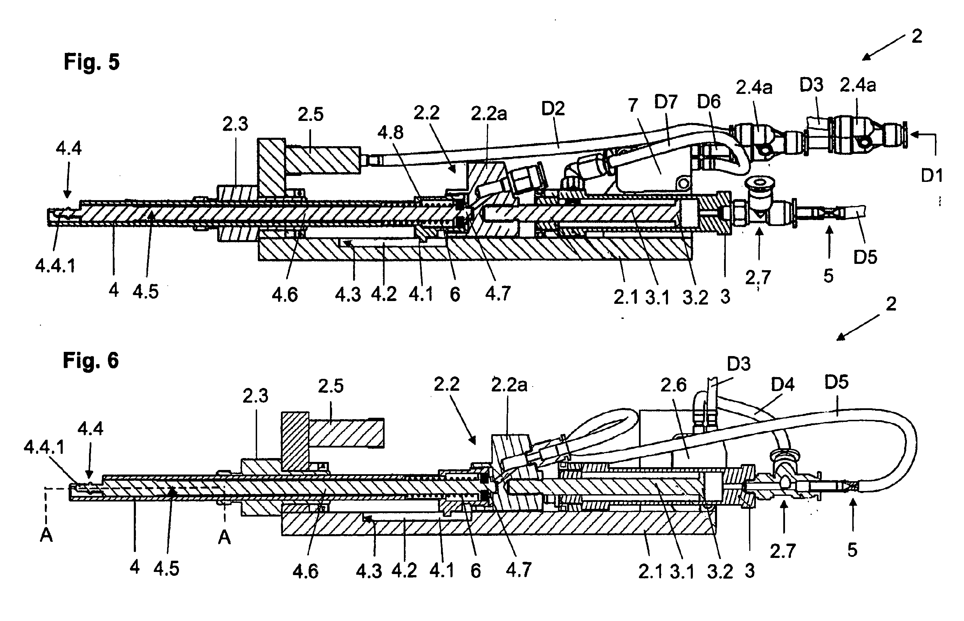

Dies wird im Folgenden unter Bezugnahme auf die Fig. 5 bis 8 näher erläutert.

In Fig. 5 und Fig. 6 sind jeweils vertikale Schnitte durch die erfindungsgemäße Vorrichtung 2 dargestellt, wobei die beiden Zeichnungen jeweils zueinander entgegen gesetzte Ansichten zeigen.

Die erfindungsgemäße Vorrichtung 2 weist zunächst ein im Wesentlichen quaderförmiges Gehäuse 2.1 (der Übersichtlichkeit wegen nur teilweise dargestellt) auf, das einerseits eine Schusseinheit 2.2, bestehend aus einem Einschusszylinder 3 und einem Schließzylinder 4 mit integrierter Sicherheitseinrichtung vor ungewolltem Auslösen, und andererseits eine Zylinderlaufbuchse 2.3, innerhalb welcher der Schließzylinder 4 geführt ist, aufweist.

Innerhalb des Einschusszylinders 3 ist ein Einschusskolben 3.1 angeordnet, welcher mittels eines Gewindes in das Verbindungsstück 2.2a der Schusseinheit 2.2 eingeschraubt ist. Weiterhin ist mit dem Verbindungsstück 2.2a ein in der Zylinderlaufbuchse 2.3 gelagerter Schließzylinder 4 verbunden angeschlossen, der zusammen mit dem Verbindungsstück 2.2a linear bewegbar ist. Dabei ist der Schließzylinder 4 mittels einer Nase 4.1 in einer Nut 4.2 in Gehäuse 2.1 geführt. Die Vorschubbewegung der Schusseinheit 2.2 wird hierbei durch einen in Gehäuse 2.1 ausgebildeten Anschlag 4.3 für die Nase 4.1 begrenzt.

Der Schließzylinder 4 weist an seinem vorderen Ende eine Clipaufnahme 4.4 auf, in der der Clip 1 insbesondere formschlüssig und angular ausgerichtet sowie positioniert gehalten wird. Wie in Fig. 5 gezeigt, kann der Clip 1 auf der Clipaufnahme 4.4, die als Fortsatz 4.4.1 über das distale Ende des Schließkolbens 4.5 hinaus ragt, aufliegen, wobei er formschlüssig gehalten wird. Zum Formschluss des Clips 1 kann dieser an seinen Zangenflanken 1.8 Noppen (nicht dargestellt) aufweisen, welche formschlüssig in entsprechende Noppen oder Vertiefungen (nicht dargestellt) einrastbar oder einsetzbar sind, welche an der Auflagefläche der Clipaufnahme 4.4 angeordnet sind.

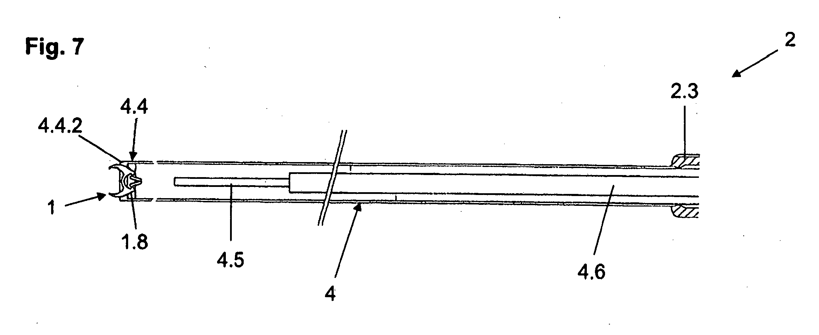

Der Clip wird auch bevorzugt seitlich gehalten, wie auch die weitere Ausführungsform der Fig. 7 zeigt, die schematisch einen Schnitt durch den vorderen Bereich der Clipaufnahme 4.4 einer alternativen Ausgestaltung entsprechend dem Schnitt A-A in der Fig. 6 darstellt. Die Fig. 7 zeigt insofern eine alternative Ausführungsform der Clipaufnahme 4.4 am distalen Ende des Schließzylinders 4. Hierbei ist der Clip 1 nicht auf einen Fortsatz 4.4.1 eines innerhalb des Schließzylinders 4 angeordneten Schließkolbens 4.5 aufgelegt oder eingestreckt, sondern in einen zangenförmigen Endbereich 4.4.2 des Schließzylinders 4 einlegbar oder einsteckbar.

Innerhalb des Schließzylinders 4 ist ein Schließkolben 4.5 angeordnet, der eine Kolbenstange 4.6 aufweist, die durch den Schließzylinder 4 auf den mittleren Bereich des Kniehebels 1.4 des Clips 1 einwirken kann und damit die unter Bezug auf die Fig. 1 bis 4 angesprochene Schließkraft für den Clip 1 ausüben kann. Im rückwärtigen Bereich des Schließkolbens 4.5 ist in einem Kolbenkopf 4.7 eine Ringdichtung eingelassen, womit der Schließkolben 4.5 dichtend innerhalb des Schließzylinders geführt ist.

Unterhalb des Kolbenkopfes 4.7 ist eine Schraubenfeder 6 angeordnet, die den Schließkolben 4.1 mit einer gewissen geringen Kraft in seiner vom Clip zurückgezogenen, in Fig. 1 dargestellten Stellung hält.

Des Weiteren weist das Gehäuse 2.1 ein Rückholmittel für die Schusseinheit 2.2 auf. Ein Rückholventil 7 (dargestellt in Fig. 5) weist einen manuellen Drehknopf (nicht dargestellt) auf, wobei eine Druckluftleitung D6 mit der Druckluftzufuhr D1 verbunden ist und eine Druckluftleitung D7 vom Rückholventil 7 in den vorderen Teil des Einschusszylinders 3 vor den Einschusskolbenkopf 3.2 führt.

Druckluft steht demgemäß ausgehend von der Druckluftzufuhr D1 permanent am Auslöseventil 2.2 (über D2), am Öffnungsventil 2.6 (über D3) und am Rückholventil 7 an.

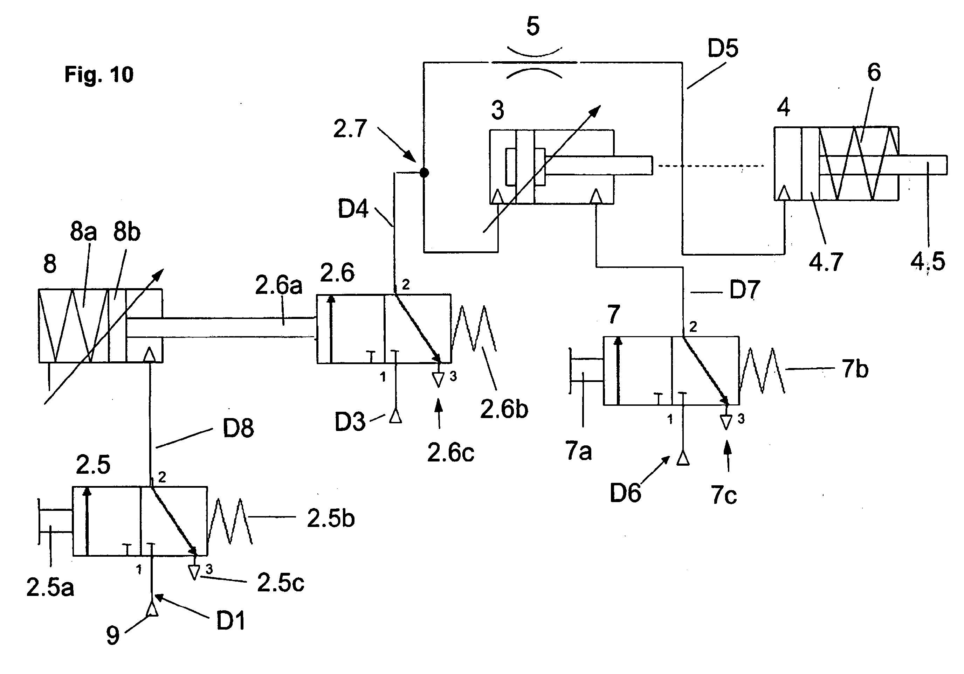

Die Druckluftzufuhr D1 führt über einen Einlaß und zwei folgende über zwei hintereinander angeordnete Y-förmige Verteiler 2.4a, 2.4b sowie weiter über einer Druckleitung D2 zu einem Auslöseventil 2.5. Das Auslöseventil 2.5 hat einen manuell betätigbaren Drehknopf (nicht dargestellt) und ist über eine weitere Druckleitung mit einem Hilfszylinder (beide in Fig. 5 ebenfalls nicht dargestellt; siehe hierzu Fig. 10) verbunden, der zur Betätigung eines weiteren Druckluftventils, einem Öffnungsventil 2.6 dient (Fig. 6). Die Druckluftzufuhr D1 ist mittels einer Druckleitung D3 eingangsseitig mit dem Öffnungsventil 2.6 verbunden (Fig. 5, 6, 10). Eine weitere Druckleitung D4 verbindet hiernach das Öffnungsventil 2.6 ausgangsseitig mit einem T-förmigen Verteiler 2.7, dessen einer Ausgang unmittelbar am rückwärtigen Ende eines Einschusszylinders 3 angeordnet ist. An einem zweiten Ausgang ist ein Reduzierstück 5 angeordnet, welches zur Druckreduzierung in einer angeschlossenen Druckleitung D5 dient, welche das Reduzierstück 5 mit einem Einlaß im Verbindungsstück 2.2a der Schusseinheit 2.2 verbindet.

Wird das Auslöseventil 2.5 geöffnet, wird die Druckluft zum erwähnten nicht dargestellten Hilfszylinder (s. hierzu Hilfszylinder 8 in Fig. 10) geleitet, der dann eine Öffnung des Öffnungsventils 2.6 bewirkt. Somit ist gewährleistet, dass sowohl bei schnellem Betätigen des Druckknopfes als auch langsameren Betätigen des manuellen Druckknopfes die Druckbeaufschlagung des Gesamtsystems gleich bleibt, da der Hilfszylinder erst ab einem gewissen einstellbaren Druck den Druckluftdurchlass freigibt. Durch das Öffnungsventil 2.6 fließt die Druckluft über die Druckleitung D4 zu einem T-förmigen Verteiler 2.7 von welchem aus die Druckluft in zwei voneinander entgegengesetzte Richtungen geleitet wird. Die Seite des T-förmigen Verteilers 2.7, die an den Einschusszylinder 3 direkt befestigt ist, führt Druckluft mit voller Druckbeaufschlagung direkt auf den im Einschusszylinder 3 laufenden Einschusskolben 3.1 und schiebt somit die gesamte Schusseinheit 2.2 nach vorne. Der Schließzylinder 4 wird hierbei durch die Zylinderlaufbuchse 2.3 geschoben. Die Bewegung erfolgt bis zu einem Anschlag 4.3, wobei die Nase 4.1 durch die Nut 4.2 geführt wird. Hierdurch wird der Clip 1 aus dem Gehäuse 2.1 heraus bewegt und wenn das Gehäuse mit seinem vorderen Ende auf Körpergewebe, gegebenenfalls unter Zwischenlage eines Netzes aufsitzt, werden die Zähne 1.2 des Clips 1 in das Gewebe eingeschossen.

Gleichzeitig mit der Druckbeaufschlagung des Einschusszylinders 3 wird die.Druckluft durch ein dem Einschusszylinder 3 entgegengesetzte Seite des Verteilers 2.7 angeordnetes durch ein Reduzierstück 5 und eine diesem angeschlossene Druckleitung D5 durch das Zwischenstück 2.2a der Schusseinheit 2.2 in den Schließzylinder 4 hinter den Kolbenkopf 4.7 des Schließkolbens 4.5 geleitet. Der Schließkolben 4.5 wird somit um eine Zeitdifferenz Δt zeitverzögert durch den Schließzylinder 4 vorgeschoben und schießt mit erheblicher Kraft gegen die Mitte des Kniehebels 1.4, wodurch auf diesen die unter Bezugnahme insbesondere der Fig. 3 und 4 beschriebene Schließkraft ausgeübt wird, der Clip 1 unter Bewegen der Zähne 1.3 gegeneinander geschlossen und schließlich der Kniehebel in seine überstreckte, in der Fig. 4 dargestellte, stabile Schließstellung gebracht wird, in der er im Gewebe gehalten wird, wodurch ein so auch gegebenenfalls zwischengelegtes Netz am Gewebe befestigt wird. In dieser Schließposition des Hebels kann dann die Vorrichtung 2 des Kniehebels 1.4 vom Clip 1 entfernt werden. Die bestimmte Zeitdifferenz Δt wird hierbei hauptsächlich bestimmt durch den Durchlass durch das Reduzierstück 5 und die Länge der Druckleitung D5 zum Verbindungsstück 2.2a der Schusseinheit 2.2.

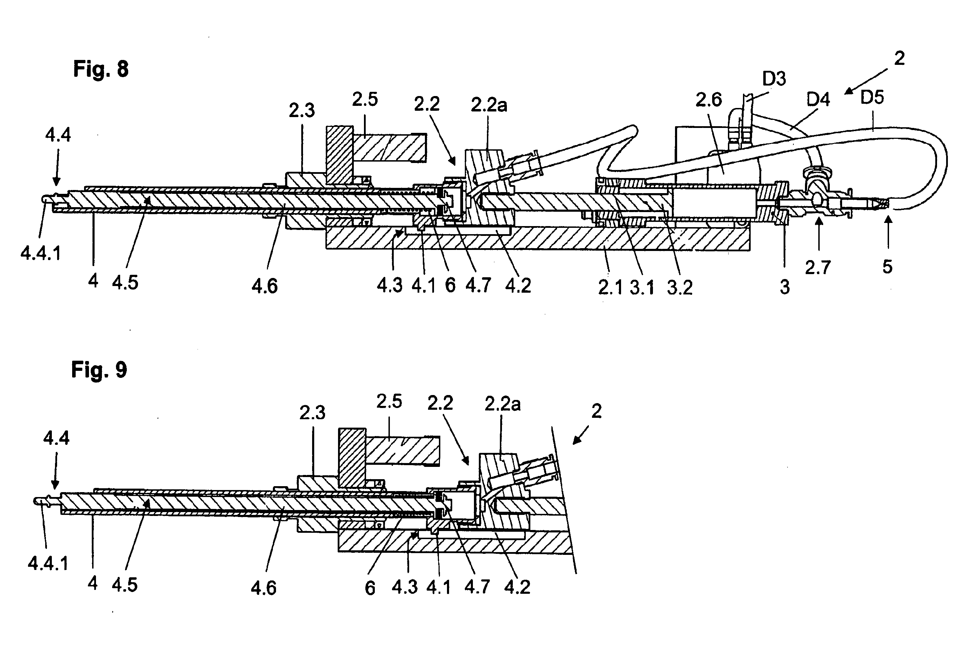

Eine Rückholung des Schließkolbens 4.5 bzw. des Kolbenkopfes 4.7 wird durch eine Schraubenfeder 5 gewährleistet, deren Vorspannung den Schließkolben 4.5 mit einer gewissen geringen Kraft in deren, in der Fig. 5 dargestellten, Ausgangsstellung zurückschiebt. In der Darstellung der Fig. 8 ist der Clip bereits in das Gewebe eingesetzt und es erfolgt eine pneumatische Rückholung des Einschusszylinders 3 und somit der kompletten Schusseinheit 2.2. Druckluft wird durch manuelles Betätigen eines Drehknopfes an einem Rückholventil 7 und der Druckluftzufuhr D1 über die Druckluftleitungen D6 und D7 innerhalb des Einschusszylinders 3 vor den Kolbenkopf 3.2 des Einschusskolbens 3.1 geleitet, mit Hilfe dessen der Einschusskolben 3.1 wieder nach hinten zurück in seine Ausgangsposition bewegt wird (siehe auch Fig.5). Die Rückwärtsbewegung des Einschusskolbens 3.1 wird durch die Abmessungen des Einschusszylinders 3 nach hinten begrenzt.

In Fig. 10 ist ein Pneumatikplan einer erweiterten Ausgestaltung der erfindungsgemäßen Vorrichtung nach DIN/ISO 1219 dargestellt und beschreibt schematisch die Leitung der Druckluft durch die erfindungsgemäße Vorrichtung. Der Pneumatikplan enthält pneumatische Schaltzeichen, die den einzelnen Komponenten der erfindungsgemäßen Vorrichtung entsprechend den Fig. 5 bis 9 zugeordnet sind und deren Bedeutung im Folgenden näher erläutert wird. Die pneumatischen Schaltzeichen zeigen die Ventile in ihrer Ruhestellung, d.h den Zustand, welchen die Ventile in einem unbetätigten Zustand einnehmen.

Der Ablauf des Pneumatikplans gliedert sich wie folgt:
- Druckluft wird von einem Auslöseventil 2.5 über einen Hilfszylinder 8 zu einem Öffnungsventil 2.6 geleitet;
- über einen T-förmigen Verteiler 2.7, welcher den Übergang des Vorschießens des gesamten Einschusszylinders 3 zu dem Vorschub des Schließkolben 4.5 in der Fig. 6 repräsentiert, wird Druckluft in den Einschusszylinder 3 geleitet;
- weiterhin wird Druckluft durch das Reduzierstück 5 zu dem Schließkolben 4 geleitet; und
- mittels des Rückholventils 7 wird Einschusszylinder 3 durch manuelles Betätigen des Drehknopfes in seine Ausgangsposition überführt.

Die Richtung der Druckluft ist im Pneumatikplan durch ein in Richtung des Druckluftstroms gerichtetes Dreieck 9 beschrieben, und zeigt somit, dass der Druckluftstrom ausgehend von der Druckluftzufuhr D1 vom Auslöseventil 2.5 zum Hilfszylinder 8 geleitet wird. Das erste Schaltzeichen im pneumatischen Ablauf der Fig. 10 ist das Auslöseventil 2.5 der Fig. 5 bis 8 und ist ein 3/2-Wegeventil mit einem Ventilkolben 2.5a und einer Rückstellfeder 2.5b. Es wird durch Muskelkraftbetätigung entgegen der Rückstellfeder 2.5b geöffnet, durch die Rückstellfeder 2.5b geschlossen und mittels einer Entlüftung 2.5c belüftet.

Dem Ventil 2.5 folgt der Hilfszylinder 8, welcher ein einfach wirkender Zylinder mit einer kolbenseitiger einstellbarer Rückstellfeder 8a und einem Zylinderkolben 8b ist, wobei ein Pfeil durch das Ventil die Einstellbarkeit der Rückstellfeder 8a bezeichnet. Die Druckluftleitung D8 leitet Druckluft von Ventil 2.5 in den vorderen federbelasteten Bereich des Hilfszylinders 8. Die Feder 8a wird mittels der Druckluft entgegen Ihrer Wirkung durch den Zylinderkolben 8b zusammengedrückt. Bei Überschreiten eines vordefinierten Öffnungsdrucks, welcher durch Einstellen der Federhärte der Feder 8a vorgegeben wird, löst die Feder 8a den Hilfszylinder 8 aus und bringt den Zylinderkolben 8b zurück in seine Ausgangsstellung. Der Zylinderkolben 8b ist verbunden mit einem Ventilkolben 2.6a des Öffnungsventils 2.6 und betätigt bei Rückgang in seine Ausgangsstellung damit das Öffnungsventils 2.6. Dieses ist ein weiteres 3/2-Wegeventil mit einer Rückstellfeder 2.6b und einer Entlüftung 2.6c, welches von dem Hilfszylinder 8 über dem manuell betätigbaren Ventilkolben 2.5a ausgelöst wird.

Der Hilfszylinder 8 und das Öffnungsventil 2.6 dienen als Schließbeschleuniger. Es soll gewährleistet sein, dass mit jeder Verwendung des Verfahrens der gleiche Druck durch die Leitungen fließen kann, der Einschusskolben 3.1 immer mit der gleichen pneumatischen Kraft vorgeschoben wird und somit den Clip 1 immer mit der gleichen Beschleunigung und Kraft in das Gewebe drückt. Die variable Muskelkraftbetätigung des Benutzers wird somit ausgeglichen, da dieser nicht bei jeder Benutzung der erfindungsgemäßen Vorrichtung mit der gleichen Stärke das Ventil auslöst.

Ein T-förmiger Verteiler 2.7 verzweigt eine von dem Öffnungsventil 2.6 kommende Druckleitung D4. Druckluft wird einerseits zu dem Einschusszylinder 3 geleitet, der ein doppeltwirkender Zylinder mit beidseitiger einstellbarer Dämpfung ist. Andererseits wird Druckluft durch ein Reduzierstück 5 mittels einer Druckleitung D5 in den rückwärtigen Teil des Schließzylinders 4 geleitet. Der Einschusszylinder 3 ist ein Zylinder mit beidseitig einstellbarer Dämpfung, welcher somit in zwei Richtungen bewegbar ist. Ein Pfeil durch das Schaltzeichen des Einschusszylinders 3 weist auf die Einstellbarkeit des Zylinders hin, da der Zylinder mit unterschiedlich starkem Druck betrieben werden kann. Druckluft gelangt von Verteiler 2.7 in den hinteren Teil des Einschusszylinders und bewirkt dessen Vorschub (siehe auch Fig. 7). Auf der gleichen Achse liegend (angedeutet durch eine gestrichelte Linie) schließt sich der Schließkolben 4.5 mit einseitiger Rückstellfeder 6 an. Hier gelangt Druckluft nach einer bestimmten zeitlichen Verzögerung, die durch den Durchlass durch das Reduzierstück, die Länge der Druckluftleitung zum Schließkolben 4.5 und den Wert der Druckluft gegeben ist, ebenfalls in den hinteren Teil des Schließkolbens 4.5 und drückt ihn entgegen der Wirkung von Feder 6 mit steigendem Druck nach vorne, wie Fig. 5 bis 8 zeigen. Nach Setzen des Clips 1 erfolgt die Rückholung des Schließkolbens 4.5 über dessen Rückstellfeder 6 bei Entspannung der davor liegenden Druckleitungen durch Entlüftung der Ventile 2.5 und 2.6. Die 3/2-Wegeventile 2.5 und 2.6 verfügen hierfür über eine zweite Ventilstellung, welche die Belüftung der Druckleitungen über die Entlüftungen 2.5c und 2.6c ermöglicht.

Das pneumatische Rückholungsmittel von dem Einschusszylinder 3 ist durch das Rückholventil 7 bezeichnet, einem 3/2-Wegeventil mit Rückstellfeder 7a und einer Entlüftung 7b, das durch Muskelkraft mittels des Ventilkolben 7c betätigt wird. Die Druckluft wird durch das Ventil 7 über die Druckluftleitung D7 in den vorderen Teil des Einschusszylinders 3 geleitet und schiebt den Einschusskolben 3.1 somit wieder in seine Ausgangsstellung zurück (siehe auch Beschreibung zu Fig. 5 bis 8). Das Rückholventil selbst wird durch eine Rückstellfeder 7a wieder in seine Ausgangsstellung gebracht. Eine Entspannung der Abluftwege des Rückholmechanismus inklusive des Einschusszylinders 3 erfolgt mittels der zweiten möglichen Ventilstellung des Druckluftrückholventils 7 über die Entlüftung 7b.

Aus vorstehendem ergibt sich, dass sobald ein Bediener - ein Arzt - das Auslöseventil 2.5 in eine Öffnungsstellung bewegt, der weitere Ablauf automatisch unter Einwirkung der freigegebenen Druckluft erfolgt und zwar in drei Stufen, indem zunächst durch den Einschusszylinder 3 der Clip 1 aus dem Gehäuse 2.1 der Vorrichtung 2 herausbewegt und in das Gewebe eingeschossen wird. In einem zweiten Schritt nach Blockieren des Einschusszylinders 3 wird durch den Anschlag 2.9 der Clip 1 unter Einwirkung des inneren Schließkolbens 4 auf die Kniehebel 1.4 geschlossen und damit am Gewebe fixiert. Als Letztes wird der Einschusskolben wieder in die Ausgangsposition durch Betätigen des Rückholventils 6 und Begrenzung der rückwärtigen Bewegung durch die Rückwand des Einschusszylinders 3 überführt.

Statt einem automatischen pneumatischen Handlungsablauf, wie er aufgrund des durch Betätigen des Druckluftventils bewirkten Auslöseimpulses bewirkt wurde, kann auch ein automatischer hydraulischer oder elektrisch oder auch elektromagnetisch gesteuerter Ablauf erfolgen, beispielsweise durch einen elektromotorischen Antrieb oder aber eine versiegelte Beaufschlagung von Elektromagneten, die zunächst den Einschusszylinder 3 und anschließend den Schließkolben 4.5 des Gehäuses 2.1 der Vorrichtung 2 nach vorne (links in den Zeichnungen) bewegen.

### Bezugszeichenliste

- 1: Clip
- 1.1: Schulter
- 1.2: Zahn
- 1.3: Spitze
- 1.4: Kniehebel
- 1.5: Filmgelenke
- 1.6: Filmgelenk
- 1.7: Ausnehmung
- 1.8: Zangenflanke
- 2: Vorrichtung
- 2.1: Gehäuse
- 2.2: Schusseinheit
- 2.3: Zylinderlaufbuchse
- 2.4a: Y-Verteiler
- 2.4b: Y-Verteiler
- 2.5: Auslöseventil
- 2.5a: Ventilkolben
- 2.5b: Rückstellfeder
- 2.5c: Entlüftung
- 2.6: Öffnungsventil
- 2.6a: Ventilkolben
- 2.6b: Rückstellfeder
- 2.6c: Entlüftung
- 2.7: T-Verteiler
- 3: Einschusszylinder
- 3.1: Einschusskolben
- 3.2: Kolbenkopf
- 4: Schließzylinder
- 4.1: Nase
- 4.2: Nut
- 4.3: Anschlag
- 4.4: Clipaufnahme
- 4.4.1: Fortsatz
- 4.4.2: Endbereich
- 4.5: Schließkolben
- 4.6: Kolbenstange
- 4.7: Kolbenkopf
- 5: Reduzierstück
- 6: Feder
- 7: Rückholventil
- 7a: Rückstellfeder
- 7b: Entlüftung
- 7c: Ventilkolben
- 8: Hilfszylinder
- 8a: Rückstellfeder
- 8b: Zylinderkolben
- 9: Richtung des Druckluftstroms
- D1: Druckluftzufuhr
- D2 - D8: Druckluftleitung

## Patentansprüche

1. Vorrichtung zum Applizieren eines medizinischen verriegelbaren Clips (1) in einen Gewebebereich, mit
a) einem von einer Ausgangsposition in Richtung des Gewebebereichs bewegbaren Vorschubmechanismus (3,3.1) mit einer Führung in Vorschubrichtung für den Clip, wobei der Clip (1) zwei in Vorschubrichtung orientierte Greifzähne (1.2) aufweist,
b) einen Anschlag (4.3) für den Vorschubmechanismus (3,3.1)
sowie
c) ein mit dem Vorschubmechanismus (3,3.1) bewegbares Verriegelungselement (4,4.5) und mit einem clipseitigem Verriegelungsmechanismus (1.1, 1.4-1.7) zur Verriegelung des Clips (1),
**dadurch gekennzeichnet, dass**
d) der Vorschubmechanismus ein automatischer ist,
e) das Verriegelungselement (4,4.5) gemeinsam und parallel zu dem Vorschubmechanismus (3,3.1) in Bewegung setzbar ist und
f) der Anschlag (4.3) die Bewegung des Verriegelungselements (4,4.5) nicht begrenzt.

2. Vorrichtung nach Anspruch 1, **dadurch gekennzeichnet, dass** die Führung eine formschlüssige und/oder reibschlüssige Aufnahme für den Clip (1) aufweist.

3. Vorrichtung nach Anspruch 1 oder 2, **dadurch gekennzeichnet, dass** der clipseitige Verriegelungsmechanismus ein Kniehebelelement (1.4) aufweist, das die Greifzähne (1.2) des Clips unter Einwirkung des Verriegelungselements gegeneinander drückt und die formschlüssige und/oder reibschlüssige Aufnahme des Clips (1) löst.

4. Vorrichtung nach einem der Ansprüche 1 bis 3, **dadurch gekennzeichnet, dass** der Vorschubmechanismus ein elektrischer oder elektromagnetischer ist.

5. Vorrichtung nach einem der Ansprüche 1 bis 3, **dadurch gekennzeichnet, dass** der Vorschubmechanismus ein pneumatischer oder hydraulischer ist.

6. Vorrichtung nach Anspruch 5, **dadurch gekennzeichnet, dass** als Auslösemechanismus eine Anordnung bestehend aus einem Auslöseventil (2.5), einem Hilfszylinder (8) und einem Öffnungsventil (2.6) vorgesehen ist.

7. Vorrichtung nach Anspruch 6, **dadurch gekennzeichnet, dass** die Druckluftzufuhr zum Vorschubmechanismus mittels des Auslöseventils (2.5), des Hilfszylinders (8) und des Öffnungsventils (2.6) erst nach Erreichen eines vorgegebenen Öffnungsdrucks pneumatisch freigebbar ist.

8. Vorrichtung nach einem der Ansprüche 1 bis 7, **gekennzeichnet durch** Rückholmittel, zur Überführung des Vorschubmechanismus (3,3.1) und des Verriegelungselements (4,4.5) in ihre Ausgangspositionen.

9. Vorrichtung nach Anspruch 8, **dadurch gekennzeichnet, dass** die Rückholmittel pneumatische Rückholkolben aufweisen.

10. Vorrichtung nach Anspruch 8, **gekennzeichnet durch** eine zwischen Vorschubmechanismus (3, 3.1) und Verriegelungselement (4,4.5) angeordnete, letztere beaufschlagende Feder (6).

11. Vorrichtung nach einem der vorgenannten Ansprüche, **dadurch gekennzeichnet, dass** der Vorschubmechanismus durch einen Einschusszylinder (3) mit einem Einschusskolben (3.1) und das Verriegelungselement durch einen Schließzylinder (4) mit einem Schließkolben (4.5) gebildet wird.

12. Vorrichtung nach Anspruch 11, **dadurch gekennzeichnet, dass** der Einschusszylinder (3) und der Schließzylinder (4) über einen T-förmigen Verteiler (2.7) und eine Druckleitung (D5) verbunden sind, wobei in der Druckleitung (D5) zum Schließzylinder (4) ein Reduzierstück (5) angeordnet ist.

13. Vorrichtung nach einem der Ansprüche 11 bis 12, **dadurch gekennzeichnet, dass** in einer Zylinderlaufbuchse (2.3) der Schließzylinder (4) läuft, innerhalb welchem der Schließkolben (4.5) geführt ist.

14. Vorrichtung nach einem der Ansprüche 5 bis 13, **dadurch gekennzeichnet, dass** die Ventile eines pneumatischen Antriebs Entlüftungen zur Entspannung der Druckleitungen aufweisen.

15. Vorrichtung nach einem der vorgenannten Ansprüche, **gekennzeichnet durch** ein Clipmagazin mit einer seriellen Clipzuführung in der Ausgangsposition.

## Claims

1. Device for the application of a medical lockable clip (1) in a tissue area, with
a) a feed mechanism (3, 3.1), which can be moved from a starting position in the direction of the tissue area, with a guide in the feed direction for the clip, wherein said clip (1) has two gripping teeth (1.2) directed in the feed direction,
b) a stop (4.3) for the feed mechanism (3, 3.1),
as well as
c) a locking element (4, 4.5), which can be moved with the feed mechanism (3, 3.1), and with a clip-side locking element (1.1, 1.4-1.7) to lock the clip (1), **characterized in that**
d) the feed mechanism is automatic,
e) the locking element (4, 4.5) can be set into motion together with and in parallel to the feed mechanism (3, 3.1) and
f) the stop (4.3) does not limit the motion of the locking element (4, 4.5).

2. Device in accordance with claim 1, **characterized in that** the guide has a positive-locking and/or frictionally engaged mount for the clip (1).

3. Device in accordance with claim 1 or 2, **characterized in that** the clip-side locking element has a toggle lever element (1.4), which presses the gripping teeth (1.2) of the clip against each other under the action of the locking element and releases the positive-locking and/or frictionally engaged mounting of the clip (1).

4. Device in accordance with one of the claims 1 through 3, **characterized in that** the feed mechanism is an electrical or electromagnetic feed mechanism.

5. Device in accordance with one of the claims 1 through 3, **characterized in that** the feed mechanism is a pneumatic or hydraulic feed mechanism.

6. Device in accordance with claim 5, **characterized in that** a device comprising a triggering valve (2.5), an auxiliary cylinder (8) and an opening valve (2.6) is provided as the triggering mechanism.

7. Device in accordance with claim 6, **characterized in that** the air supply to the feed mechanism can be pneumatically released by means of a triggering valve (2.5), the auxiliary cylinder (8) and the opening valve (2.6) only after a preset opening pressure has been reached.

8. Device in accordance with one of the claims 1 though 7 , **characterized by** a restoring means for moving the feed mechanism (3, 3.1) and the locking element (4, 4.5) into their respective starting positions.

9. Device in accordance with claim 8, **characterized in that** the restoring means have pneumatic restoring pistons.

10. Device in accordance with claim 8, **characterized by** a spring (6), which is arranged between the feed mechanism (3, 3.1) and the locking element (4, 4.5) and acts on the latter.

11. Device in accordance with one of the above claims, **characterized in that** the feed mechanism is formed by a shoot-in cylinder (3) with a shoot-in piston (3.1) and the locking element is formed by a closing cylinder (4) with a closing piston (4.5).

12. Device in accordance with claim 11, **characterized in that** the shoot-in cylinder (3) and the closing cylinder (4) are connected via a T-shaped distributor (2.7) and a pressure line (D5), wherein a reducer (5) is arranged in the pressure line (D5) leading to the closing cylinder (4).

13. Device in accordance with one of the claims 11 or 12, **characterized in that** the closing cylinder (4), within which the closing piston (4.5) is guided, runs in a cylinder liner (2.3).

14. Device in accordance with one of the claims 5 through 13, **characterized in that** the valves of the pneumatic drive have vent holes to release the pressure in the pressure lines.

15. Device in accordance with one of the above claims, **characterized by** a clip magazine with a serial clip feed in the starting position.

## Revendications

1. Dispositif pour appliquer une pince médicale verrouillable (1) dans une partie d'un tissu, avec :
a) un mécanisme d'avancement (3, 3.1) pouvant être déplacé, en partant d'une position de départ, en direction de la partie d'un tissu, avec un guide s'étendant dans la direction d'avancement pour la pince, la pince (1) comportant deux dents de préhension (1.2) orientées dans la direction d'avancement ;
b) une butée (4.3) prévue pour le mécanisme d'avancement (3, 3.1) ; et
c) un élément de verrouillage (4, 4.5) mobile pourvu du mécanisme d'avancement (3, 3.1) et avec un mécanisme de verrouillage (1.1, 1.4 - 1.7) placé du côté de la pince pour verrouiller la pince (1) ;
**caractérisé en ce que** :
d) le mécanisme d'avancement est un mécanisme automatique ;
e) l'élément de verrouillage (4, 4.5) peut être mis en mouvement conjointement avec le mécanisme d'avancement (3, 3.1) et parallèlement à lui ; et
f) la butée (4.3) ne délimite pas le mouvement de l'élément de verrouillage. (4, 4.5).

2. Dispositif selon la revendication 1, **caractérisé en ce que** le guide comporte un logement réalisé par complémentarité de formes et/ou par complémentarité de frottements pour la pince (1).

3. Dispositif selon la revendication 1 ou 2, **caractérisé en ce que** le mécanisme de verrouillage placé du côté de la pince comporte un élément de genouillère (1.4) comprimant les dents de préhension (1.2) de la pince les unes contre les autres sous l'effet de l'élément de verrouillage et desserre le logement réalisé par complémentarité de formes et/ou par complémentarité de frottements de la pince (1).

4. Dispositif selon l'une quelconque des revendications 1 à 3, **caractérisé en ce que** le mécanisme d'avancement est un mécanisme électrique ou électromagnétique.

5. Dispositif selon l'une quelconque des revendications 1 à 3, **caractérisé en ce que** le mécanisme d'avancement est un mécanisme pneumatique ou hydraulique.

6. Dispositif selon la revendication 5, **caractérisé en ce que** le mécanisme de déclenchement utilisé comporte un agencement composé d'une soupape de déclenchement (2.5), d'un cylindre auxiliaire (8) et d'une soupape d'ouverture (2.6).

7. Dispositif selon la revendication 6, **caractérisé en ce que** l'amenée d'air comprimé conduisant au mécanisme d'avancement est libérée par voie pneumatique à l'aide de la soupape de déclenchement (2.5), du cylindre auxiliaire (8) et de la soupape d'ouverture (2.6) après avoir atteint une pression d'ouverture prédéfinie.

8. Dispositif selon l'une quelconque des revendications 1 à 7, **caractérisé par** la présence de moyens de rappel servant à transférer le mécanisme d'avancement (3, 3.1) et l'élément de verrouillage (4, 4.5) dans leur position de départ.

9. Dispositif selon la revendication 8, **caractérisé en ce que** les moyens de rappel comportent des pistons de rappel pneumatiques.

10. Dispositif selon la revendication 8, **caractérisé par** la présence d'un ressort (6) disposé entre le mécanisme d'avancement (3, 3.1) et l'élément de verrouillage (4, 4.5) et sollicitant ce dernier.

11. Dispositif selon l'une quelconque des revendications précédentes, **caractérisé en ce que** le mécanisme d'avancement est formé par un cylindre d'impact (3) doté d'un piston d'impact (3.1) et que l'élément de verrouillage est formé par un cylindre de fermeture (4) doté d'un piston de fermeture (4.5).

12. Dispositif selon la revendication 11, **caractérisé en ce que** le cylindre d'impact (3) et le cylindre de fermeture (4) sont reliés via un répartiteur (2.7) en forme de T et une conduite de pression (D5), une pièce de réduction (5) étant disposée dans la conduite de pression (D5) en direction du cylindre de fermeture (4).

13. Dispositif selon l'une quelconque des revendications 11 à 12, **caractérisé en ce que** le cylindre de fermeture (4) s'étend dans une chemise de cylindre (2.3) à l'intérieur de laquelle le piston de fermeture (4.5) est guidé.

14. Dispositif selon l'une quelconque des revendications 5 à 13, **caractérisé en ce que** les soupapes d'un entraînement pneumatique comportent des purges servant à libérer la pression régnant dans les conduites de pression.

15. Dispositif selon l'une quelconque des revendications précédentes, **caractérisé par** la présence d'un chargeur de pince avec amenée de pince en série dans la position de départ.
